Europäisches Patentamt

European Patent Office

Office européen des brevets

(11) Numéro de publication: **0 084 013**
**A2**

(12) **DEMANDE DE BREVET EUROPEEN**

(21) Numéro de dépôt: **83400074.7**

(22) Date de dépôt: **12.01.83**

(51) Int. Cl.³: **C 12 N 15/00**
**C 12 N 5/00, C 12 P 1/00**
**G 01 N 33/54**
**//C12R1/91**

(30) Priorité: **13.01.82 FR 8200476**

(43) Date de publication de la demande:
**20.07.83 Bulletin 83/29**

(84) Etats contractants désignés:
**AT BE CH DE FR GB IT LI LU NL SE**

(71) Demandeur: **UNIVERSITE PIERRE ET MARIE CURIE (PARIS VI)**
**4, place Jussieu**
**F-75005 Paris(FR)**

(71) Demandeur: **INSTITUT PASTEUR**
**28, rue du Docteur Roux**
**F-75724 Paris Cedex 15(FR)**

(72) Inventeur: **Soriano, Luis**
**12 rue Poulletier**
**F-75004 Paris(FR)**

(72) Inventeur: **Bricout, Fernand**
**5, avenue du Colonel Bonnet**
**F-75016 Paris(FR)**

(72) Inventeur: **Fortier, Bernard**
**19, rue Jean Daudin**
**F-75015 Paris(FR)**

(72) Inventeur: **Kemler, Rolf**
**Alexander Strasse 63**
**D-7400 Tubingen(DE)**

(74) Mandataire: **Phélip, Bruno et al,**
**c/o Cabinet Harlé & Phélip 21, rue de La Rochefoucauld**
**F-75009 Paris(FR)**

(54) **Lignées cellulaires hybrides murines anti-herpès, procédé d'obtention, anticorps monoclonaux anti-herpès non neutralisants, applications biologiques.**

(57) L'invention concerne un procédé pour l'obtention de lignées cellulaires hybrides murines produisant des anticorps monoclonaux anti-Herpès non neutralisants.

Ce procédé consiste à effectuer la fusion de splénocytes de souris immunisées contre un virus Herpès et de cellules de myélome de souris.

L'invention concerne également les anticorps produits par ces lignées.

Application: diagnostic des affections virales causées par les virus Herpès simplex type 1 et type 2.

Lignées cellulaires hybrides murines anti-Herpès,
procédé d'obtention, anticorps monoclonaux anti-
Herpès non neutralisants, applications biologiques.

La présente invention concerne des lignées cellulaires hybrides murines anti-Herpès et un procédé
pour leur obtention. Elle concerne également les anticorps monoclonaux anti-Herpès non neutralisants produits par ces lignées cellulaires hybrides ainsi que
les applications de ces anticorps monoclonaux non
neutralisants au diagnostic des affections virales
causées par de tels virus, notamment par les virus
Herpès simplex chez l'homme.

Le traitement efficace des affections virales
nécessite la mise au point, par les virologistes,
de méthodes de diagnostic rapides, qui permettent
d'une part d'affirmer, avec certitude et en un laps
de temps court, la présence d'un virus dans les
lésions observées et d'autre part, de déterminer le
traitement approprié, alors que les lésions virales
sont encore minimes. C'est notamment le cas des virus
Herpex simplex de type 1 et de type 2 (couramment
dénommés par les abréviatins HSV-1 et HSV-2), vis-
à-vis desquels des médicaments, tels que la cytosine
arabinoside ou l'acycloguanosine peuvent être prescrits.
Le diagnostic rapide des affections causées par les
virus Herpès simplex est particulièrement justifié
dns les cas suivants :

    - de l'herpès néo-natal, qui d'une simple éruption vésiculeuse, dégénère en une encéphalite généralement mortelle ou laisse des séquelles neurologiques
définitives ;

    - de l'encéphalite herpétique qui, en l'absence
de traitement, évolue soit vers la mort, soit vers
des séquelles très graves ;

    - de la kérato-conjonctivite herpétique au cours

6682 FR

de laquelle l'atteinte cornéenne entraîne généralement une cécité définitive.

Dans ces trois cas, un traitement thérapeutique
approprié, effectué dans les heures qui suivent
l'apparition des premiers symptômes, est indispensable pour éviter les séquelles provoquées par ces
affections virales. Il est donc très important de
pouvoir rapidement déceler le virus responsable de
l'affection pour déterminer le traitement thérapeutique approprié.

D'autres applications du diagnostic rapide de
l'Herpès simplex sont également intéressantes, notamment dans le cadre de la prévention de l'Herpès néo-
natal et dans le cadre des transplantations d'organes.

En effet, la prévention de l'Herpès néo-natal
nécessite :

- l'éradiction de la transmission sexuelle de
l'Herpès type 2 : ce qui nécessite un dépistage
facile de cette maladie en dermato-vénérologie,

- la détermination d'un groupe de femmes à haut
risque, qui est réalisée par un dépistage systématique, par des techniques de haute sensibilité
(par exemple par dosage immunoenzymatique), de la
présence de l'Herpès simplex type 1 ou type 2 dans les
voies génitales.

D'autre part, le peu de spécificité des lésions
pose également un problème aux dermatologistes ,
notamment dans le cadre des transplantations d'organes où la gravité de l'évolution clinique conduit
à traiter précocement les patients.

La gravité des affections herpétiques et les
possibilités thérapeutiques actuelles exigent désormais le développement de méthodes de haute sensibilité et de grande rapidité pour le diagnostic de
ce virus.

Pour répondre à ces exigences, on a jusqu'à présent utilisé principalement des sérums animaux (par exemple des sérums de lapins ou de cobayes), qui ont permis le développement de procédés rapides de dosage, tels que l'immunofluorescence, l'immunoenzymologie et la radioimmunologie.

Cependant, de tels sérums sont des mélanges d'anticorps qui, en dépit d'absorptions répétées, révèlent de nombreux déterminants antigéniques; des réactions croisées vis-à-vis d'autres virus (notamment du groupe Herpès) ainsi que de structures cellulaires (cellules nerveuses notamment) rendent parfois le diagnostic aléatoire.

On a déjà décrit certains types d'anticorps monoclonaux anti-Herpès simplex. A cet effet, on peut citer :

- l'article de PEREIRA et al. dans "Infection and Immunity " Août 1980 p. 724-732. Les anticorps décrits dans cet article sont des anticorps monoclonaux anti-Herpès simplex type 1 qui sont neutralisants, c'est-à-dire qu'ils neutralisent le pouvoir infectant des souches Herpès Simplex type 1 ;

- l'article de ZWEIG et al. dans "Journal of Virology" Nov. 1979 p. 676-678 qui décrit la production d'anticorps monoclonaux contre les protéines nucléocapsidiques des virus Herpès simplex type 1 ou type 2. Ces anticorps reconnaissent exclusivement les protéines p 40 et p 45 qui sont contenues dans les nucléocapsides des virus Herpès simplex type 1 ou type 2.

- l'article de BALACHANDRAN et al. qui décrit des anticorps monoclonaux spécifiques glycoprotéines du virus Herpès simplex type 2.

- l'article de SEVIER et al. dans Clin. Chem. 27/11 1797-1806 (1981) qui est relatif à l'utilisation des anticoprs monoclonaux en immunologie clinique. Un nombre important d'anticorps monoclonaux sont cités parmi lesquels figurent les anticorps monoclonaux anti-Herpès simplex neutralisants selon PEREIRA, cités ci-dessus.

On a maintenant trouvé des lignées cellulaires hybrides murines produisant des anticorps monoclonaux anti-Herpès simplex non neutralisants qui possèdent un titre élevé et une spécificité rigoureuse. De tels anticorps permettent du fait de leur spécificité rigoureuse et de leur grande affinité de détecter en l'espace de quelques heures, à moindre frais et en toute certitude, des quantités très faibles d'antigènes herpétiques.

Les lignées cellulaires hybrides selon l'invention sont obtenues par fusion de splénocytes de souris immunisées contre le virus de l'Herpès simplex et de cellule de myélome de souris.

La fusion de cellules de myélome de souris avec des lymphocytes de souris immunisées a été proposée pour la première fois en 1975 par KOHLER et MILSTEIN [Nature 256 495-497 (1975)]. La technique de fusion, également dénommée "technique des hybridomes" a été depuis largement utilisée pour la production d'anticorps monoclonaux purs. Cependant, jusqu'à présent, on n'a jamais, à la connaissance de la demanderesse, fabriqué des anticorps anti-Herpès simplex non neutralisants par une telle technique. De plus, il faut noter que l'on ne peut pas savoir avec certitude si l'on obtiendra par cette technique de fusion l'anticorps ayant la spécificité désirée, même si cette technique est connue dans son principe, du fait, notamment que chaque antigène a une constitution propre et un anticorps spécifique correspondant.

Jusqu'à présent, la technique de préparation d'antigènes viraux et la qualité des souches n'ont permis d'obtenir qu'un nombre relativement réduit de clônes produisant les anticorps spécifiques recherchés.

La présente invention concerne plus particulièrement des lignées cellulaires hybrides murines anti-Herpès simplex, dénommés "hybridines murines anti-Herpès simplex" qui produisent des anticorps monoclonaux anti-Herpès simplex non neutralisants.

Sous son aspect général, le procédé selon l'invention pour l'obtention de lignées cellulaires hybrides murines anti-Herpès simplex comprend les étapes suivantes :

1) d'immunisation de souris avec un virus Herpès simplex (voie IV et IP)

2) de prélèvement de la rate et des adénopathies mésentériques des souris immunisées et séparation de splénocytes ;

3) de fusion des splénocytes ainsi obtenus avec des cellules de myélome de souris en présence d'un promoteur de fusion ;

4) de culture des cellules hybrides obtenues dans un milieu sélectif sur lequel ne se développent pas les cellules de myélomes non fusionnées, et en présence d'éléments nutritifs appropriés ;

5) de sélection des cellules produisant l'anticorps désiré et clonage de ces cellules.

Les souches virales utilisées pour l'immunisation des souris sont des souches de virus Herpès simplex, telles que par exemple la souche VR 3 (ATCCVR 539) pour obtenir des anticorps monoclonaux anti-Herpès simplex type 1 et la souche G (ATCCVR 734) pour fournir des anticorps monoclonaux anti-Herpès simplex types 1 et 2. Les souches VR3 et G sont décrites dans les documents ci-après :

- souche VR3 : (MAC INTYRE) SCHMIDT and LENNETTE: J. IMMUNOL 1961, 86, 137

- souche G : EJERCITO, P.M., KIEFF E.D.; ROIZMAN B. J. gen. Virol 1968 $\underline{2}$ 357-364.

Les souches virales sont cultivées sur un milieu nutritif approprié, par exemple des fibroblastes de lapin permettant d'obtenir un titre très élevé de particules virales. Après purification des virus en gradient de saccharose effectuée de manière classique les antigènes viraux ainsi obtenus sont stockés à - 80°C par exemple selon la méthode de Ben-Porat T and KAPLAN AS Virology 1970, 265-273.

Le protocole d'immunisation des souris et la quantité d'antigènes viraux utilisés doivent être suffisants pour obtenir une quantité appropriée de splénocytes immunisés.

Avec des souris Balb/C on a trouvé que le protocole d'immunisation ci-après était approprié :

- injection au jour $J_o$ de 80 µg d'antigène dans du sérum physiologique (0,5 ml) par voie intra-péritonéale ;

- injection au jour $J_o$ + 30 de 80 µg d'antigène dans du sérum physiologique (0,5 ml) par voie intra-veineuse.

Trois jours après le rappel, c'est-à-dire au jour $J_o$ + 33, les souris sont sacrifiées et leur rate et ganglions mésentériques sont aussitôt prélevés , lavés dans du liquide de Hank et dilacérés. Les splénocytes sont ensuite séparés des lymphocytes par des moyens classiques, par exemple par centrifugation, puis lavés avec le même milieu et numérés.

Parallèlement, des cellules de myélome de souris Sp 2/o [SHULMAN, M, C.D WILDE and G. KOLHER 1978 Nature $\underline{276}$, 269] cultivées en milieu DULBECCO (vendu par Flow Laboratories) sont lavées dans du milieu de Hank et également numérés.

La fusion des cellules, qui constitue la seconde étape du procédé de l'invention consiste à mélanger des cellules de la rate, à savoir les splénocytes ainsi que les lymphocytes provenant des adénopathies mésentériques des souris immunisées contre les virus Herpès simplex, avec des cellules de myélome de souris, avantageusement à raison de 1 splénocyte pour 2 cellules de myélome (rapport optimum).

La fusion proprement dite consiste à mélanger dans un tube les splénocytes et les cellules de myélome dans les proportions indiquées ci-dessus, en présence d'un promoteur de fusion classique. A titre de tel promoteur on peut citer les polyéthylèneglycols, par exemple le polyéthylène-glycol 4000 qui possède un poids moléculaire de 4000 [FAZEKAS de St GROTH S., D. SCHEIDEGGER, J. of IMMUNOLOGICAL METHODS 1980, 35, p. 1-21].

Après incubation pendant environ 1 minute à environ 37°C, on dilue le culot cellulaire résultant dans un milieu approprié, en opérant de manière à ne pas détruire les cellules, et on procède à la culture initiale des cellules hybrides résultantes dans un milieu sélectif qui n'est pas favorable aux cellules de myélome qui n'ont pas fusionné, c'est-à-dire aux cellules de myélome initiales, de telle sorte que seulement les cellules hybrides se reproduiront alors que les cellules de myélome meurent. Les cellules de myélome sont dépourvues de l'enzyme hypoxanthine-guanine-phosphorybosyl-transférase et ne supportent pas les milieux couramment dénommés milieux HAT, c'est-à-dire les milieux contenant de l'hypoxanthine, de l'aminoptérine et de la thymidine.

Aux fins de l'invention on utilise avantageusement comme milieu sélectif le milieu DULBECCO contenant 15 % de sérum de veau foétal et additionné de thymidine $(1,6. 10^{-5}M)$ d'hypoxanthine $(10^{-4}M)$, d'ami-

noptérine $(4.10^{-7}M)$ (Milieu DULBECCO HAT).

Dans ce milieu sélectif sont ajoutés des éléments nutritifs appropriés pyruvate + 1-glutamine. On utilise avantageusement selon l'invention les macrophages péritonéaux de souris non immunisées. Les cultures sont réalisées à 37°C environ dans une étuve contenant 10 % de $CO_2$.

Dans ces conditions on obtient au bout d'un certain temps uniquement des cellules hybrides ; en effet, étant donné le milieu sélectif utilisé les cellules de myélome non fusionnées ne survivent pas, pour les raisons indiquées ci-dessus. D'autre part, les splénocytes qui n'auraient pas fusionné donnent seulement un nombre limité de générations, puis meurent. Seules les cellules hybrides, qui possèdent à la fois le caractère maligne des cellules de myélome et l'aptitude des splénocytes à se développer sur le milieu sélectif choisi se reproduisent.

On procède ensuite à la sélection des cellules hybrides produisant l'anticorps désiré et on clône les cellules hybrides sélectionnées sur un milieu de culture approprié, DULBECCO + Hypoxanthine + Aminoptérine + Thymidine. On utilise avantageusement le milieu de culture utilisé pour la culture initiale des cellules hybrides. Comme pour la culture initiale on opère à 37°C environ et en présence de 10 % de $CO_2$.

La sélection est effectuée par méthode immunoenzymatique et immunofluorescence indirecte à partir des surnageants des cultures de cellules. Seules sont retenues lors de chaque clonage, les cellules secrétant les anticorps caractéristiques détectés par les deux méthodes ci-dessus, qui seront décrites plus en détail ci-après au sujet de l'application des anticorps monoclonaux obtenus.

On a constaté, en recherchant un éventuel pouvoir de neutralisation des ascîtes, que les anticorps monoclonaux selon l'invention sont des anticorps non neutralisants.

Selon le procédé de l'invention on obtient des lignées de cellules hybrides murines anti-Herpès simplex humains type 1 ou types 1 et 2 selon le virus utilisé pour immuniser les souris.

Ces lignées de cellules hybrides ont été déposées le 8 janvier 1982 dans la Collection Nationale de Culture de Microorganismes de l'Institut Pasteur à Paris sous les numéros I-182 et I-183 qui sont respectivement :

n° I-183 (08-01-82) : lignée de cellules hybrides murines anti-Herpès simplex de type 1

n° I-182 (08-01-82) : lignée de cellules hybrides murines anti-Herpès simplex de types 1 et 2.

On les conserve par congélation dans 90 % de sérum foetal et 10 % de diméthylsufloxyde (DMSO).

Ces lignées cellulaires hybrides secrètent des anticorps monoclonaux anti-Herpès simplex type 1 ou communs aux types 1 et 2, qui peuvent être caractérisés de la façon suivante :

- ils sont spécifiques soit de l'Herpès simplex de type 1, soit des antigènes communs aux deux types d'Herpès. Ces anticorps monoclonaux font également partie de l'invention. On les conserve dans les conditions ci-après :

- congélation à - 80°C

- ou lyophilisation.

On a trouvé que l'anticorps monoclonal anti-Herpès simplex de type 1 non neutralisant selon l'invention est capable par immunofluorescence indirecte ou par méthode immunoenzymatique de détecter de façon spécifique le virus Herpès simplex de type 1.

Par contre, l'anticorps monoclonal anti-Herpès simplex de type 1 et 2 non neutralisant selon l'invention est capable de détecter, par ces deux mêmes méthodes, les deux types de virus Herpès simplex (type 1 et type 2).

Ces anticorps monoclonaux non neutralisants peuvent convenir particulièrement bien pour la détection de virus Herpès simplex notamment par les techniques d'immunofluorescence (directe ou indirecte) ou immunoenzymatique.

L'immunofluorescence observée avec les anticorps monoclonaux selon l'invention est essentiellement cytoplasmique et non pas intra-nucléaire comme c'est le cas avec les anticorps selon ZWEIG et al. cités précédemment.

Les anticorps selon l'invention peuvent également être utilisés dans d'autres techniques, telles que les techniques radioimmunologiques.

On rappellera ci-après le mode opératoire de ces méthodes de dosage bien connues de l'homme de l'art :

Dosage par immunofluorescence

Les cellules provenant du grattage des lésions (ou de l'empreinte de la biopsie) sont fixées à l'acétone sur une lame.

L'anticorps est mis en contact avec les cellules, par exemple pendant 1/2 heure à 37°C.

Après lavage, une anti IgG de souris marquée à la fluoresceine est mise en contact avec les cellules, incubée comme précédemment puis lavée.

La lame est examinée avec un microscope à U.V., les cellules parasitées par l'Herpès apparaissent fluorescentes.

En alternative on peut marquer directement l'anticorps spécifique anti-Herpès simplex et supprimer l'utilisation de l'anti-IgG.

Dosage immunoenzymatique

Il consiste dans un premier temps à absorber les antigènes présents dans/la lésion sur des anticorps spécifiques préalablement fixés sur un support (plaque, billes ou similaires), puis à mettre en présence ce support avec les anticorps spécifiques marqués par une enzyme, enfin à rechercher l'activité enzymatique par une réaction colorée lue soit à l'oeil, soit avec un photomètre. La durée d'un tel dosage est d'environ quatre heures.

L'invention concerne également les coffrets de diagnostic pour méthode de dosage par immunofluorescence ou immunoenzymatique.

Le coffret de diagnostic par immunofluorescence directe selon l'invention contient :

- au moins un anticorps monoclonal anti-Herpès simplex selon l'invention/marqué avec un agent de fluorescence, tel que la fluoresceine,

- des lames pour observation au microscope

- des tampons.

Ce coffret convient pour une détection par immunofluorescence directe. On peut également réaliser avec les anticorps monoclonaux selon l'invention des dosages par immunofluorescence indirecte (dosage par double anticorps) c'est-à-dire en utilisant un second anticorps, généralement des anticorps anti-IgG de souris marqués à la fluorescéine.

Ainsi, l'invention, a également pour objet un coffret de dosage par immunofluorescence indirecte qui contient :

- au moins un anticorps monoclonal anti-Herpès simplex non neutralisant

　　　　- un anticorps anti-IgG de souris marqué avec
un agent de fluorescence, tel que la fluoresceine,
　　　　- des lames pour observation au microscope
　　　　- des tampons.

Le coffret de diagnostic par méthode immunoenzymatique selon l'invention contient :
　　　　- au moins un anticorps monoclonal anti-Herpès
non neutralisant marqué par une enzyme,
　　　　- un support immunoadsorbant,
　　　　- un agent révélateur de l'activité enzymatique
　　　　- des tampons.

Les coffrets selon l'invention contiennent
avantageusement deux anticorps monoclonaux anti-
Herpès simplex non neutralisants, à savoir un anticorps monoclonal anti-Herpès simplex type 1 et un
anticorps monoclonal anti-Herpès simplex types 1
et 2. De tels coffrets permettent de détecter la
présence dans une affection virale de l'un des virus
Herpès simplex type 1 ou type 2.

Les anticorps monoclonaux selon l'invention peuvent également être utilisés dans d'autres procédés
de diagnostic, tels que par exemple les procédés
ci-après :

1) dosage par immuno-microscopie électronique
où les particules virales sont concentrées par piégeage des anticorps monoclonaux et visualisées par
coloration négative. La durée d'un tel dosage est
d'environ une heure.

2) agglutination de staphylocoques dorés préalablement sensibilisés par l'anticorps monoclonal.
La durée de cette détermination est d'environ 30
minutes.

Les anticorps monoclonaux selon l'invention sont
un progrès très important pour la virologie clinique.
De tels anticorps, du fait de leur spécificité rigoureuse et de leur grande affinité permettent de détecter en l'espace de quelques heures, à moindres frais et en toute certitude des quantités très faibles d'antigène ; un diagnostic aussi sûr n'était pas réalisable jusqu'à présent avec les sérums hyperimmuns. L'introduction des anticorps monoclonaux anti-Herpès simplex est donc une amélioration très nette pour la virologie médicale qui débouche sur de nombreuses applications cliniques (gynéco-obstétrique, néo-natalogie, dermatologie, neuro-chirurgie, ophtalmalogie, etc...).

Ainsi, grâce aux anticorps selon l'invention on peut détecter rapidement la présence des virus Herpès simplex sur des prélèvements de vésicules (en dermatologie), une biopsie encéphalique (en neurochirurgie) les prélèvements faits par grattages conjonctivaux (en ophtalmologie), grattages au niveau du col (en gynécologie) et grattages des vésicules sur le nouveau-né (en néo-natalogie).

L'invention va être maintenant décrite plus en détail par les exemples illustratifs mais non limitatifs ci-après :

EXEMPLE 1:

Fabrication de lignées de cellules hybrides murines anti-Herpès simplex type 1 et type 2.

Les virus Herpès simplex type 1 et type 2 seront dénommés ci-après HSV-1 et HSV-2.

1) Protocole d'immunisation

On a utilisé les souches virales suivantes :

a) - souche VR 3 pour les cellules hybrides HSV-1
    - souche G    pour les cellules hybrides HSV-2

Les souches ont été cultivées sur fibroblastes de lapin.

Après purification des virus en gradient de saccharose, les antigènes viraux ainsi obtenus ont été stockés à - 80°C.

b) Immunisation des souris
-------------------------------
Balb/C Les souris/ ont été immunisées soit vis-à-vis de HSV-1, soit vis-à-vis de HSV-2 selon le protocole suivant :

- 80 µg d'antigène par voie intra-péritonéale au jour $J_o$,

- 80 µg en intra-veineuse au jour $J_o$ + 30.

2) Protocole de fusion

a) Les cellules
------------------

3 jours après le rappel (jour $J_o$ + 33) les souris ont été sacrifiées, la rate et les ganglions mésentériques ont été aussitôt prélevés, lavés dans du liquide de Hank et dilacérés. Les splénocytes et les lymphocytes ont été séparés par centrifugation puis lavés dans le même liquide et numérés.

De même les cellules de myélome Sp2/0 qui avaient été cultivées en milieu DULBECCO ont été lavées dans du Hank  et numérées.

Parallèlement les macrophages péritonéaux ont été obtenus, par lavage du péritoine de souris Balb/C non immunisées avec du sérum physiologique.

b) la fusion
----------

Pour obtenir la formation des cellules hybrides, les splénocytes et les Sp2/0 ont été mélangés dans un tube à raison de 1 splénocyte pour 2 Sp2/0 , soit pour une plaque de micro-culture de 96 cupules $50.10^6$ splénocytes pour $100.10^6$ Sp2/0.

Après centrifugation, le culot cellulaire a été remis en suspension dans 0,5 ml de polyéthylène-glycol 4000 à 50 % en $H_2O$.

Après une incubation de 1 minute à 37°C, 10 ml de liquide de Hank ont été rajoutés lentement :

1 goutte toutes les 10 secondes pendant 5 minutes,
puis le reste du liquide de Hank en agitant très doucement l'ensemble.

3) la culture initiale

La culture initiale a été réalisée en plaque
de micro-culture (Costar, 96 cupules) en présence
de milieu de DULBECCO contenant 15 % de sérum de veau
foetal et additionné de thymidine ($1,6.10^{-5}$M), d'hypoxanthine ($10^{-6}$M), d'aminoptérine ($4.10^{-7}$M) (milieu
DULBECCO HAT).

Dans chaque cupule ont été successivement versés
50 $\mu$l d'une suspension contenant $3;10^3$ macrophages
péritoneaux, puis 150 $\mu$l contenant $5.10^5$ splénocytes
et donc $10^6$ Sp2/0.

Trois plaques ont ainsi été réalisées à partir
de chaque souris testée.

Les plaques ont été immédiatement mises à 37°C
dans une étuve contenant 10 % de $CO_2$.

4) Technique de sélection des cellules hybrides

Au cours des différentes étapes de sélection
des cellules hybrides, le protocole ci-après a été
utilisé :

Un volume de surnageant de culture a été prélevé
stérilement (0,15 ml) et remplacé par un même volume
de milieu neuf.

Chaque surnageant ainsi prélevé a été ensuite
testé par la technique immunoenzymatique bien connue
sous le nom de technique ELISA (qui est une technique
de dosage immunoenzymatique avec immunoadsorbant),
en micro-technique, vis-à-vis de 3 antigènes différents : Herpès 1 (souche VR 3), Herpès 2 (souche G)
et antigène témoin (cellules Hela ATCC CCL 2 - Journal
of Exp. Med. 1953 vol. 97 p. 695).

Les surnageants ont été étudiés à une seule dilution (1/4). La présence d'anticorps de souris a été recherchée par un Fab de mouton anti IgG (H + L)
de souris (Institut Pasteur Production).

Parallèlement chaque surnageant positif a également été testé en immunofluorescence indirecte vis-à-vis de 4 souches d'Herpès de type connu : 2 HSV-1 et 2-HSV-2 provenant d'isolements à partir de lésions (dilution au 1/10).

Seules ont été retenues lors de chaque clonage, les celullules sécrétant des anticorps caractéristiques détectés par la technique ELISA et par immunofluorescence indirecte.

Chaque clone a été repliqué lentement puis congelé dans 90 % de sérum de veau foetal et 10 % de DMSO.

Des sous-clonages des hybrides ont été réalisés en plaque de micro-culture.

Chaque cupule a d'abord reçu une suspension de $3.10^3$ macrophages péritonéaux de souris (en DULBECCO HAT) puis statistiquement 3 ou 1 cellules d'hybridome dans le même milieu.

Les plaques ont été alors incubées à 37°C en présence de 10 % de $CO_2$.

Les cupules contenant un clone ont été testées comme précédemment vis-à-vis des antigènes HSV-1 et HSV-2 tant par technique immunoenzymatique que par immunofluorescence.

Deux clonages successifs ont ainsi été réalisés.

EXEMPLE 2 :

Mise en évidence de la spécificité des clones HSV-1 et HSV-2 obtenus.

A - activité vis-à-vis des virus HSV-1 et HSV-2
-------------------------------------------------

Les deux clones obtenus ont été testés aux dilutions suivantes : 1/1, 1/10 et 1/100 par immunofluorescence indirecte vis-à-vis des souches Herpès simplex type 1 : souches VR3, A 44 et B, Herpès simplex type 2 : souches G et MS.

D'autres souches provenant de malades et typées par immunofluorescence (sérums DAKO) [Ref VESTERGAARD B.F., THYBO H. scand. J. of Infect. Dis. 1974, 6, 113] ont été testées avec ces surnageants (8 souches étaient de type 1, et 8 souches de type 2).

On a opéré de la façon suivante :

Dans des lames spéciales stériles et avec des alvéoles, on a déposé une goutte d'un prélèvement viral ; on a fait sécher sur ventilateur (30°C),puis on a plongé la lame dans de l'acétone à - 20°C. Les lames ainsi obtenues peuvent être soit stockées à - 80°C jusqu'à utilisation ou utilisées immédiatement pour la détection.

On a déposé 20 μl d'anticorps monoclonal (surnageant) dans du PBS (solution tampon phosphate) dans chaque alvéole et on a incubé 30 minutes à 37°C, puis on a lavé avec du PBS PH 7,4 trois fois. On a ensuite incubé avec des anticorps anti-IgG de souris marqués à la fluorescéine, 30 minutes à 37°C. On a ensuite lavé avec du PBS pH 7,4 trois fois, puis avec de l'eau distillée (en quelques secondes). On a monté les lames avec de la glycérine tamponnée avec PBS pH 7,4 et on a procédé à la lecture au microscope de fluorescence.

Dans tous les cas des résultats comparables ont été obtenus :

- Clone HSV 1, fluorescence positive vis-à-vis de seules souches HSV-1 jusqu'à dilution 1/10.

- Clone HSV-2, fluorescence dans les mêmes conditions vis-à-vis des souches HSV-1 et HSV-2.

B - Comportement vis-à-vis des virus non HSV
-------------------------------------------------

On a opéré comme ci-dessus en utilisant des sou-

ches de virus non HSV, à savoir de grippe A, grippe B, adenovirus, respiratoire syncitial, cytomegalovirus.

Aucune fluorescence n'a été observée avec les clones HSV-1 et HSV-2 non dilués.

Ce résultats montrent que les anticorps mono-clonaux secrétés par les cellules hybrides de l'invention présentent une haute spécificité vis-à-vis de virus Herpès simplex 1 et 2.

C - Recherche du pouvoir neutralisant des ascites
-------------------------------------------------

La recherche d'une neutralisation du pouvoir infectant des souches d'Herpès simplex (HSV) a été réalisée sur cellules Vero cultivées en plaques de microtitration en présence de milieu DULBECCO conte-mant 1 % de sérum de veau foetal.

Les souches virales étaient pour le type 1 la souche VR-3 (ATCC VR 539) et pour le type 2 la souche G (ATCC VR 734).

Chaque ascite a été diluée de 1/2 en 1/2 à partir de la dilution du 1/5 dans le même milieu que précédemment. Chaque dilution ainsi obtenue a été mélangée avec un même volume d'une dilution de la souche virale étudiée (titre 200 DI 50 % pour 50 microlitres). Après une incubation de 1/2 heure à 37°C, chaque mélange ainsi obtenu a été réparti sous un volume de 50 microlitres dans 12 cupules contenant une couche uniforme de cellules Vero et un volume de 50 microlitres du milieu de culture.

Les plaques ainsi obtenues ont été cultivées trois jours à 37°C en présence de 5 % de $CO_2$.

La présence d'un foyer de cellules présentant un effet cytopathogène a été considérée comme correspondant à une absence de neutralisat de la souche étudiée et les résultats étudiés selon la méthode statistique de Kärber.

Chaque ascite a été étudiée du 1/5 à 1/640.

Aucune cupule n'a présenté une quelconque neutralisation.

| DILUTION DES ASCITES: | 1/5 | 1/10 | 1/20 | 1/40... | 1/640 |
|---|---|---|---|---|---|
| ASCITE ANTI HSV-1 | 0 | 0 | 0 | 0 | 0 souche VR3 |
| | 0 | 0 | 0 | 0 | 0 souche G |
| ASCITE ANTI HSV 1 et 2 | 0 | 0 | 0 | 0 | 0 souche VR3 |
| | 0 | 0 | 0 | 0 | 0 souche G |

0 : absence totale de neutralisation dans les 12 cupules étudiées.

Aucune des deux ascites étudiées ne présentait un pouvoir neutralisant vis-à-vis des deux souches étudiées.

Immunoprécipitation :

Par cette méthode on a déterminé le poids moléculaire des produits des cellules infectées par HSV-1 et HSV-2 qui ont été immunoprécipités par les anticorps monoclonaux selon l'invention.

Le mode opératoire était le suivant :

On a utilisé des cellules Vero, des cellules Vero infectées par HSV-1 et des cellules Vero infectées par HSV-2.

Le marquage des cellules a été effectué avec de la $S^{35}$ méthionine pendant 16 heures (20 pCi/ml de milieu) dans du milieu Eagle modifié par un milieu DULBECCO, ne contenant pas de méthionine mais contenant 10 % de sérum de veau foetal.

Les cellules ont été lavées trois fois dans du PBS (tampon phosphate) ne contenant pas d'ions $Ca^{+++}$ et $Mg^{++}$, lysées dans un tampon contenant 0,5 % de NP-40 et 0,5 % de Triton 100 (NP-40 et Triton 100 sont des détergents) pendant 30 minutes à 4°C. On a utilisé un lysat cellulaire de $2 \times 10^{6}$ cpm pour chaque essai. On a ajouté à chaque lysat cellulaire

150 μl de surnageant du milieu de culture contenant l'anticorps monoclonal selon l'invention ; on a maintenu à 4°C pendant une heure et on a ensuite ajouté 75 μg d'anticorps de lapin anti-IgG de souris purifiés sur colonne d'affinité. Le milieu résultant ainsi obtenu a été maintenu pendant 30 minutes à 4°C et on y a ensuite ajouté des billes de Sepharose-Protéine A (disponibles dans le comerce). Les billes ont ensuite été lavées cinq fois par des moyens classiques et les immuno-complexes formés ont été analysés sur un gel de polyacrylamide dénaturant dans du sodium dodécylsulfate, tel que décrit par LAEMMLI. Les immunoprécipités ont été examinés par autoradiographie.

Résultats :

- avec l'anticorps monoclonal anti-HSV-1 obtenu selon l'exemple 1, on a observé une bande correspondant à des poids moléculaires d'environ 80.000 seulement avec des cellules infectées avec HSV-1 et rien avec les cellules infectées avec HSV-2 ;

- avec l'anticorps monoclonal anti HSV-1 et 2 obtenu selon l'exemple 1, on a observé trois bandes correspondant à des poids moléculaires compris entre 75 000 et 98 000 avec les cellules infectées avec HSV-1 et sept bandes correspondant à des poids moléculaires compris entre 44 000 et 110 000.

Cet essai d'immunoprécipitation confirme que l'on obtient selon l'invention un anticorps monoclonal anti-HSV-1 capable de détecter de façon spécifique le virus Herpès simplex type 1, tandis que l'anticorps monoclonal anti-HSV-1 et 2 est capable de détecter les types 1 et 2 du virus Herpès simplex.

EXEMPLE 3 :

Dosage immunoenzymatique utilisant un anticorps monoclonal selon l'invention

Pour effectuer un tel dosage, on a procédé de la façon suivante :

Les anticorps monoclonaux ont été dilués dans du tampon Tris-EDTA-NaCl pH 9,6. On a coulé les anticorps monoclonaux sur une plaque NUNC (96 trous) 200 $\mu$l/trou, et incubé à 4°C 1 nuit ou plus. On a ensuite lavé la plaque d'eau déminéralisée avec 0,05% Tween 20. On a déposé la suspension à analyser, 200 $\mu$l/trou. On a incubé pendant 3 heures à 37°C, puis lavé avec du tampon. On a ensuite incubé 200 $\mu$l d'anticorps monoclonaux marqués à la péroxydase, puis procédé à un lavage. On a ensuite utilisé de l'orthophénylène diamine (OPD) dans le tampon citrate-phosphate pH 5,0 pour révéler l'activité enzymatique, que l'on observe à l'oeil nu ou au photomètre.

## REVENDICATIONS

1. Procédé pour l'obtention de lignées cellulaires hybrides murines produisant des anticorps monoclonaux anti-Herpès non neutralisants, caractérisé en ce qu'il consiste à effectuer la fusion de splénocytes de souris immunisées contre un virus Herpès et de cellules de myélome de souris et à sélectionner les cellules hybrides produisant les anticorps désirés.

2. Procédé selon la revendication 1 pour l'obtention de lignées cellulaires hybrides murines produisant des anticorps monoclonaux anti-Herpès simplex humains, caractérisé en ce qu'il comprend les étapes suivantes :

1) immunisation de souris avec un virus Herpès simplex,

2) prélèvement de la rate et des adénopathies mésentériques des souris immunisées et séparation des splénocytes ;

3) fusion des splénocytes ainsi obtenus avec des cellules de myélome de souris en présence d'un promoteur de fusion ;

4) culture des cellules hybrides obtenues dans un milieu sélectif sur lequel ne se développe pas les cellules de myélome non fusionnées, et en présence d'éléments nutritifs appropriés ;

5) sélection des cellules produisant l'anticorps non neutralisant désiré et clonage de ces cellules.

3. Procédé selon la revendication 2, caractérisé en ce que :

1) l'immunisation des souris est réalisée par deux injections à 30 jours d'intervalle, la première par voie intrapéritonéale, la seconde par voie intraveineuse,

2) la fusion est réalisée en utilisant un splénocyte pour 2 cellules de myélome en présence de polyé- thylèneglycol 4000,

3) la culture des cellules hybrides est réalisée sur un milieu sélectif contenant de l'hypoxanthine, de l'aminoptérine et de la thymidine à 37°C en présence de 10 % de $CO_2$ et de macrophages péritonéaux de souris non immunisés comme éléments nutritifs.

4) la sélection des souches hybrides est effectuée par dosage par immunofluorescence indirecte et dosage immunoenzymatique.

5) le clonage est réalisé à 37°C en présence de 10 % de $CO_2$.

4. Procédé selon l'une quelconque des revendications 1 à 3, caractérisé en ce que l'immunisation des souris est réalisée avec le virus Herpès simplex type 1, souche ATCC VR 539.

5. Procédé selon l'une quelconque des revendications 1 à 3, caractérisé en ce que l'immunisation des souris est réalisée avec le virus Herpès simplex type 2, souche ATCC VR 734.

6. Lignées de cellules hybrides murines produisant des anticorps monoclonaux anti-Herpès simplex humains obtenues par le procédé selon l'une quelconque des revendications 1 à 5.

7. Lignée de cellules hybrides murines anti-Herpès simplex type 1 déposée à la Collection Nationale de Cultures de microorganismes de l'Institut Pasteur-Paris sous le n° I-183 08-01-82.

8. Lignée de cellules hybrides murines anti-Herpès simplex type 1 et 2, déposée à la Collection Nationale de Cultures de microorganismes de l'Institut Pasteur-Paris sous le n° I-182 08-01-82.

9. Anticorps monoclonaux anti-Herpès simplex non neutralisants produits par la lignée de cellules hybrides murines anti-Herpès simplex obtenues par le procédé selon l'une quelconque des revendications 1 à 5.

10. Anticorps monoclonal anti-Herpès simplex type 1 non neutralisant produit par la lignée des cellules hybrides murines anti-Herpès type 1 déposée à la Collection Nationale de Cultures de microorganises de l'Institut Pasteur-Paris, sous le n° I- 183 08-01-82.

11. Anticorps monoclonal anti-Herpès simplex types 1 et 2 non neutralisant, produit par la lignée de cellules hybrides murines anti-Herpès simplex types 1 et 2 déposée à la Collection Nationale de Cultures de microorganismes de l'Institut Pasteur-Paris, sous le n ° I-182 08-01-82.

12. Application des anticorps monoclonaux selon l'une quelconque des revendications 9 à 11, au diagnostic rapide des affections virales causées par les virus Herpès notamment par les virus Herpès simplex humains.

13. Application selon la revendication 12, caractérisée en ce que le diagnostic est réalisé par immunofluorescence ou par méthode immunoenzymatique.

14. Coffret de diagnostic des virus Herpès simplex par immunofluorescence directe, caractérisé en ce qu'il comprend :

- au moins un anticorps monoclonal anti-Herpès simplex marqué avec un agent de fluorescence, tel que la fluoresceine,

- des lames pour observation au microscope,

- des tampons.

15. Coffret de diagnostic des virus Herpès simplex par immunofluorescence indirecte caractérisé en ce qu'il comprend :

- au moins un anticorps monoclonal anti-Herpès non neutralisant

- un anticorps anti-IgG de souris marqué avec un agent de fluorescence tel que la fluorescéine,

- des lames pour observation au microscope

- des tampons

16. Coffret de diagnostic des virus Herpès simplex par méthode immunoenzymatique, caractérisé en ce qu'il comprend :

- au moins un anticorps monoclonal anti-Herpès non neutralisant marqué par une enzyme,

- éventuellement un support immunoadsorbant,

- un agent révélateur de l'activité enzymatique

- des tampons.

17. Coffret selon l'une quelconque de revendications 14 à 16 pour le diagnostic des virus Herpès simplex type 1 et type 2, caractérisé en ce qu'il contient :

- un anticorps monoclonal anti-Herpès simplex type 1 selon la revendication 10,

- un anticorps monoclonal anti-Herpès simplex types 1 et 2 selon la revendication 11.